# EUROPEAN PATENT SPECIFICATION

(11) **EP 4 041 089 B1**
(45) Date of publication and mention of the grant of the patent: **08.04.2026**
(21) Application number: 20786542.9
(22) Date of filing: 06.10.2020
(51) Int. Cl.: A61B 8/12, A61B 8/00

(54) **DISPOSABLE INTRALUMINAL SENSING DEVICE WITH USAGE TRACKING**
WEGWERFBARE INTRALUMINALE SENSORVORRICHTUNG MIT BENUTZUNGSVERFOLGUNG
DISPOSITIF DE DÉTECTION INTRALUMINAL JETABLE PRÉSENTANT UN SUIVI D'UTILISATION

(30) Priority: 08.10.2019 US 201962912345 P
(43) Date of publication of application: 17.08.2022
(73) Proprietor: Koninklijke Philips N.V., 5656 AG Eindhoven (NL)
(72) Inventor: MANIAN, Suresh, Renganathan, 5656 AE Eindhoven (NL)
(74) Representative: Philips Intellectual Property & Standards
(86) International application number: PCT/EP2020/077970
(87) International publication number: WO 2021/069424

(56) References cited:
- WO-A1-2019/076971
- WO-A1-2019/077141
- US-A1- 2015 289 944

## Description

### TECHNICAL FIELD

The present disclosure relates generally to instrumented intraluminal sensing devices, and in particular, to intraluminal imaging catheters, such as intra-cardiac echocardiography (ICE) catheters, that are equipped with usage tracking hardware.

### BACKGROUND

Diagnostic and therapeutic ultrasound catheters have been designed for use inside many areas of the human body. In the cardiovascular system, a common diagnostic ultrasound method is intraluminal ultrasound imaging, with intra-cardiac echocardiography (ICE) being a specific example of intraluminal imaging. Typically, a single rotating transducer or an array of transducer elements is used to transmit ultrasound at the tips of the catheters. The same transducers are used to receive echoes from the tissue. A signal generated from the echoes is transferred to a console which allows for the processing, storing, display, or manipulation of the ultrasound-related data.

Intraluminal imaging catheters, such as ICE catheters (*e.g.,* Siemens Acunav, St. Jude ViewFlex), are generally used to image heart and surrounding structures, for example, to guide and facilitate medical procedures, such as transseptal lumen punctures, left atrial appendage closures, atrial fibrillation ablation, and valve repairs. Commercially-available ICE catheters have distal ends which can be articulated by a steering mechanism located in a handle at the proximal end of the catheter. For example, an intraluminal imaging catheter such as an ICE catheter may be inserted through the femoral or jugular vein when accessing the anatomy, and steered in the heart to acquire images necessary to the safety of the medical procedures.

Intraluminal imaging catheters may be reusable or disposable. It is technically possible to reuse disposable intraluminal catheters, a process over which manufacturers have little or no control. Multiple uses could degrade the performance of the catheter. This could adversely impact images the clinician is using to make diagnostic and/or therapeutic decisions, and can thus put patients' health at risk. Care must also be taken to ensure proper sterilization and sterile repackaging techniques are employed prior to reuse, patients' health may also be at risk. Clinicians may be unaware that a catheter is used rather than new, or may be aware that a catheter has been used previously, but unaware of the number of previous uses or the conditions of use or reprocessing.

WO 2019/076971 discloses a disposable catheter with a memory storing information such as catheter type, serial number, configuration for sensitivity, channel matching etc.

US 2015/289944 discloses a disposable intravascular device with a limited number of uses. It has a memory storing information such as a serial number, configuration information, initial parameters, reuse instructions, manufacturing data etc.

### SUMMARY

The invention is defined by the claims. An ultrasound imaging system includes a usage tracking disposable intraluminal catheter with a non-volatile memory. The intraluminal catheter communicates with a manufacturer's workstation capable of writing data to the non-volatile memory, a clinical workstation that operates the catheter to acquire medical data from a patient while positioned within the patient, and a reprocessor's workstation capable of writing encrypted data to regions of the non-volatile memory. The system keeps track of the number of times a given catheter is authorized to be used, the number of times it has been used, and the maximum duration of an allowed use to ensure the device is properly reprocessed between uses. The catheter's memory is subdivided so that the reprocessor's workstation only has the ability to modify a limited portion of the memory. The system relies on data stored within the catheter itself, and on information and software stored and operating on a remote server accessible via a web application, mobile application, or similar remote access architecture. The present disclosure advantageously provides mechanisms for accurately tracking the use, reprocessing, and reuse of the catheter, and therefore provides a solution to the problems discussed above.

One general aspect includes an intraluminal imaging catheter, including: a flexible elongate member configured to be positioned within a body lumen of a patient; an ultrasound transducer array coupled to the flexible elongate member and configured to obtain imaging data while positioned within the body lumen; and a memory comprising a plurality of regions, wherein each of the plurality of regions is configured to store one or more values for one or more fields, wherein the plurality of regions comprises a first region, wherein the one or more fields in the first region comprises at least one of a device identification, a serial number, or a manufacturing date, wherein the plurality of regions comprises at least a second region, wherein the one or more fields in the at least the second region comprises a current use count, a maximum number of uses, and a status of the intraluminal imaging catheter.

A system can include the intraluminal imaging catheter and one or processor circuits communicatively coupled to the intraluminal imaging catheter. The one or more processor circuits can be part of one or more computers. The processor circuit(s) and/or computer(s) can be configured to perform particular operations or actions by virtue of having software, firmware, hardware, or a combination of them installed that in operation causes or cause the processor circuit(s) and/or computer(s) to perform the actions. One or more computer programs can be configured to perform particular operations or actions by virtue of including instructions that, when executed by data processing apparatus, cause the apparatus to perform the actions. Implementations of the described techniques may include hardware, a method or process, or computer software on a computer-accessible medium.

Implementations may include one or more of the following features. The one or more fields in the second region comprises a single usage time period and a beginning timestamp of current use. The one or more fields in the first region comprises two or more of the device identification, the serial number, or the manufacturing date. The plurality of regions further comprises a third region, wherein the one or more fields in the third region comprises the status of the intraluminal imaging catheter, and wherein the one or more fields in the second region comprises the current use count and the maximum number of uses. The one or more values in the second region and the one or more values in the third region are encrypted differently. The one or more values in the second region and the one or more values in the third region are encrypted based on the one or more values in the first region. The one or more values for the status of the intraluminal imaging catheter in the third region comprises: an unlocked value representative of authorization for the intraluminal imaging catheter to be used while connected to a clinical console; an in use value representative of an initial connection of the intraluminal imaging catheter to the clinical console; or a locked value representative of a subsequent connection of the intraluminal imaging catheter to the clinical console. The one or more values for the status of the intraluminal imaging catheter in the third region comprises: a permanently locked value representative of a connection the intraluminal imaging catheter to the clinical console when use of the intraluminal imaging catheter exceeds the maximum number of uses. The intraluminal imaging catheter can include: a communication cable terminating at a connector, wherein the memory is disposed within the connector; and a handle configured to control at least one of a position or an orientation of the ultrasound transducer array within the body lumen, wherein the handle is coupled to the communication cable and the flexible elongate member, and wherein the ultrasound transducer array is configured for intracardiac echocardiography (ICE).

One general aspect includes an intraluminal imaging system, including: an intraluminal imaging catheter, including: a flexible elongate member configured to be positioned within a body lumen of a patient; an ultrasound transducer array coupled to the flexible elongate member and configured to obtain imaging data while positioned within the body lumen; and a non-volatile memory; and one or more processor circuits in communication with the intraluminal imaging catheter and configured to operate the ultrasound transducer array to obtain the imaging data; and write information into the non-volatile memory including: a number of times the intraluminal imaging catheter may be used; a number of times the intraluminal imaging catheter has been used; a status of the intraluminal imaging catheter, the status including at least one of locked, unlocked, or in-use, where the one or more processor circuits will not operate the ultrasound transducer array if the status of the intraluminal imaging catheter is locked, or the number of times the intraluminal imaging catheter has been used is equal to or greater than the number of times the intraluminal imaging catheter has been used. Other embodiments of this aspect include corresponding computer systems, apparatus, and computer programs recorded on one or more computer storage devices, each configured to perform the actions.

Implementations may include one or more of the following features. The intraluminal imaging catheter where the one or more processor circuits is configured to set the status of the intraluminal imaging catheter to: in-use while the intraluminal imaging catheter is being used, locked after use of the intraluminal imaging catheter, or unlocked during reprocessing of the intraluminal imaging catheter. The intraluminal imaging catheter where the information written by the one or more processor circuits into the non-volatile memory further includes a checksum. The intraluminal imaging catheter where at least a portion of the information written by the one or more processor circuits into the non-volatile memory is encrypted. The intraluminal imaging catheter where the information written by the one or more processor circuits into the non-volatile memory further includes: a maximum duration for each use of the intraluminal imaging catheter; a time stamp indicating a start time of a use of the intraluminal imaging catheter. The intraluminal imaging catheter where the one or more processor circuits includes a timer. The intraluminal imaging catheter where once a difference between the timer and the time stamp equals or exceeds the maximum duration, the one or more processor circuits is configured to change the status of the intraluminal imaging catheter to locked. The intraluminal imaging catheter where, when the intraluminal imaging catheter is in use and the difference between the timer and the time stamp is less than the maximum duration, the intraluminal imaging catheter is configured to be repeatedly connected to and disconnected from a clinical workstation without triggering the one or more processor circuits to change the status of the intraluminal imaging catheter from in-use to locked. Implementations of the described techniques may include hardware, a method or process, or computer software on a computer-accessible medium.

One general aspect includes an intraluminal imaging system, including: an intraluminal imaging catheter, including: a flexible elongate member configured to be positioned within a body lumen of a patient; an ultrasound transducer array coupled to the flexible elongate member and configured to obtain imaging data while positioned within the body lumen; and a non-volatile memory configured for communication with a manufacturer's workstation including a first processor circuit, a clinical workstation including a second processor circuit, and a reprocessor's workstation including a third processor circuit, such that: the manufacturer's workstation writes into the non-volatile memory a status value of unlocked and a number of times the intraluminal imaging catheter may be used, and the clinical workstation operates the intraluminal imaging catheter and writes into the non-volatile memory. The intraluminal imaging system also includes a usage counter that increments every time the intraluminal imaging catheter is operated; a status value of in-use that replaces the status value of unlocked while the intraluminal imaging catheter is being operated; a status value of locked that replaces the status value of in-use when a given time has elapsed since a start of the intraluminal imaging catheter being operated. The reprocessor's workstation writes into the intraluminal imaging catheter a status value of unlocked that replaces either or both of the status value of in-use or the status value of locked. The clinical workstation will not operate the intraluminal imaging catheter if the status value of locked is in the non-volatile memory, or the usage counter contains a value larger than the number of times the intraluminal imaging catheter may be used. Other embodiments of this aspect include corresponding computer systems, apparatus, and computer programs recorded on one or more computer storage devices, each configured to perform the actions of the methods.

Implementations may include one or more of the following features. The intraluminal imaging system further including the manufacturer's workstation. The intraluminal imaging system further including the clinical workstation. The intraluminal imaging system further including the reprocessor's workstation, where the reprocessor's workstation further includes a license status value selected from licensed or not-licensed. The intraluminal imaging system further including a remote server in communication with the reprocessor's workstation, where the remote server is configured to be access via an application executed the third processor circuit of the reprocessor's workstation. Implementations of the described techniques may include hardware, a method or process, or computer software on a computer-accessible medium.

The ultrasound imaging system has particular but not exclusive application in intra-cardiac echo cardiography. Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Figure 1** is a schematic diagram of an example imaging system according to embodiments of the present disclosure.
**Figure 2** is a perspective view of an example imaging assembly positioned for coupling to a catheter according to embodiments of the present disclosure.
**Figure 3** shows the proximal portion of an example catheter cable attached to a connector at the proximal portion of the catheter.
**Figure 4** is a schematic view of an example reprocessing workstation according to at least one embodiment of the present disclosure.
**Figure 5** is a schematic view of an example information flow between a manufacturing workstation, a clinical workstation, and a reprocessing workstation, according to at least one embodiment of the present disclosure.
**Figure 6** is a flow diagram indicating exemplary movements of a usage tracking, disposable intraluminal imaging catheter, and the device information stored thereon, according to at least one embodiment of the present disclosure.
**Figure 7** is a more detailed flow diagram indicating exemplary process steps enacted upon a usage tracking, disposable intraluminal imaging catheter through multiple use and reprocessing cycles, according to at least one embodiment of the present disclosure.
**Figure 8** shows an exemplary initial state of the non-volatile memory of a usage tracking, disposable intraluminal catheter when it leaves the manufacturer, according to at least one embodiment of the present disclosure.
**Figure 9** shows an exemplary state of the non-volatile memory of a usage tracking, disposable intraluminal catheter according to at least one embodiment of the present disclosure.
**Figure 10** shows an exemplary state of the non-volatile memory of a usage tracking, disposable intraluminal catheter according to at least one embodiment of the present disclosure.
**Figure 11** shows an exemplary state of the non-volatile memory of a usage tracking, disposable intraluminal catheter according to at least one embodiment of the present disclosure.
**Figure 12** shows an exemplary state of the non-volatile memory of a usage tracking, disposable intraluminal catheter according to at least one embodiment of the present disclosure.
**Figure 13** shows an exemplary state of the non-volatile memory of a usage tracking, disposable intraluminal catheter according to at least one embodiment of the present disclosure.
**Figure 14** shows an exemplary state of the non-volatile memory of a usage tracking, disposable intraluminal catheter according to at least one embodiment of the present disclosure.
**Figure 15** is a schematic diagram of a processor circuit, according to embodiments of the present disclosure.

### DETAILED DESCRIPTION

Disclosed is an ultrasound imaging system that includes an intraluminal imaging catheter, a non-volatile memory, a manufacturer's console or manufacturer's workstation capable of writing encrypted or unencrypted data to three different regions of the non-volatile memory, a clinical console or clinical workstation capable of writing encrypted data to two of the three regions of the non-volatile memory, and a reprocessor's console or reprocessor's workstation capable of writing encrypted data to one of the three regions of the non-volatile memory. The system keeps track of the number of times a given catheter is authorized to be used, the number of times it has been used, the maximum duration of an allowed use, and other related information as necessary to prevent unauthorized reuse and to ensure the device is properly reprocessed between uses.

Dividing the non-volatile memory into at least two regions (e.g., three different regions: one public read-only area and two encrypted and potentially checksumed read-write areas) allows for a secure implementation of a reprocessing/reuse technique. This is because the reprocessor is able to change a portion of the memory (e.g., to change a status variable from "In-Use" or "Locked" to "Unlocked") to advantageously permit reuse, but is only able to modify that specific portion of memory, advantageously ensuring security so that the device is not reused more times than the manufacturer determines is safe. Encryption and checksumming of the read/write areas prevents unauthorized users from accessing or altering the information, thereby ensuring that the device is not reused without proper reprocessing by an authorized party. When implemented across a manufacturer's workstation, clinical workstation, reprocessor's workstation, and on the sensor device itself, the system permits convenient reuse of medical sensors in a secure, trackable, verifiable manner.

It is generally challenging to encourage clinical users to update the software on a clinical intraluminal imaging workstation, and the system therefore relies on data stored within the catheter itself, and on information and software stored and operating on a remote server accessible via a web application, mobile application, or similar remote access architecture.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the ICE system is described in terms of intraluminal imaging, it is understood that it is not intended to be limited to this application. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

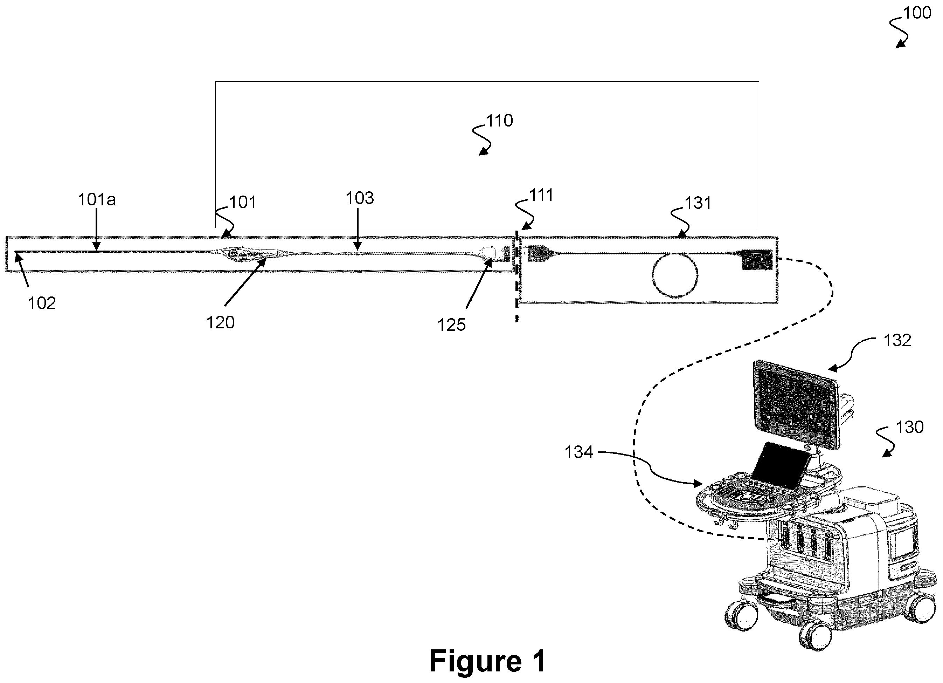

**Figure 1** is a schematic diagram of an example imaging system 100 according to embodiments of the present disclosure. The system 100 may include an intraluminal ultrasound imaging device 110, a control and processing system 130 (for example, a console including a computer), and a patient interface module (PIM) 131 extending between the device 110 and the control and processing system 130.

The ultrasound imaging device 110 may include a catheter 101. The catheter 101 may include one or more flexible elongate members sized and shaped, structurally arranged, and/or otherwise configured to be positioned within a body lumen of a patient. In some embodiments, the catheter 101 includes an ultrasound imaging assembly 102, a catheter body or shaft 101a, a communication cable 103, a handle 120, and a connector 125. In some embodiments, the catheter body/shaft 101a and catheter communication cable 103 may be referred to as a flexible elongate member. The catheter shaft 101a is sized and shaped, structurally arranged and/or otherwise configured to be positioned within a body lumen of a patient (*e.g.,* vasculature such as blood vessels or chambers of the heart). Respective portions of the catheter communication cable 103 extend within the catheter shaft 101a, the handle 120, and the connector 125. The imaging assembly or probe tip 102 may be attached it a distal end of the catheter shaft 101. The catheter shaft 101a may include a lumen that the catheter communication cable 103 may pass through. The proximal end of the catheter shaft 101a may be attached to the control handle 120, for example, by a resilient strain reliever. The control handle 120 may be used for manipulation of the ultrasound imaging device 110 and manual control of the ultrasound imaging device 110. The ultrasound imaging device 110 may include an imaging assembly 102 with ultrasound transducer elements and associated circuitry. The handle 120 may include actuators, a clutch or brake, and other steering control components for steering the ultrasound imaging device 110.

The catheter communication cable 103 may pass through one or more of the catheter shaft 101a, handle 120, and connector 125. In some embodiments, during assembly, the catheter cable 103 The catheter communication cable 103 may be electrically and mechanically coupled to the imaging assembly 102 and may include a plurality of electrical wires.

The cable 103 may be configured to provide suitable configurations for interconnecting the clinical control and processing system 130 and the monitor 132 to the imaging assembly 102. The clinical control and processing system 130 may be used for processing, storing, analyzing, and manipulating data, and the monitor 132 may be used for displaying obtained signals generated by the imaging assembly 102. The clinical control and processing system 130 (also referred to herein as a control and processing system, a processing system, a clinical workstation, or base station) can include one or more processors, memory, one or more input devices, such as keyboards and any suitable command control interface device. The control and processing system 130 may be operable to facilitate the features of the intraluminal imaging system 100 described herein. For example, a processor can execute computer readable instructions stored on a non-transitory tangible computer readable medium. The monitor 132 may be any suitable display device, such as organic light emitting diode (OLED), liquid-crystal display (LCD) panel or the like, and may comprise a touchscreen interface.

In operation, a physician or a clinician may advance the catheter 101 into a lumen, such as a blood vessel, body lumen, or portion of a heart anatomy. By manipulating controls on the handle 120, the physician or clinician may steer the catheter 101 to a position near the area of interest to be imaged. The imaging process may include activating the ultrasound transducer elements on the imaging assembly 102 to produce ultrasonic energy. A portion of the ultrasonic energy is reflected by the area of interest and the surrounding anatomy, and the ultrasound echo signals are received by the ultrasound transducer elements. The cable 103 may be used to transfer the received echo signals to the control and processing system 130 where the ultrasound image is reconstructed and displayed on the monitor 132. In some embodiments, the processing system 130 can control the activation of the ultrasound transducer elements and the reception of the echo signals. In some embodiments, the control and processing system 130 and the monitor 132 may be part of a same system.

While some embodiments of the present disclosure refer to an imaging device, an ultrasound imaging device, or an intraluminal imaging device, it is understood that the ultrasound imaging device 110 and the system 100 generally may be used to image vessels, structures, lumens, and/or any suitable anatomy/tissue within a body of a patient including any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the imaging device 110 may be used to examine man-made structures such as, but without limitation, heart valves, stents, shunts, filters and other devices. For example, the ultrasound imaging device 110 can be positioned within fluid filled or surrounded structures, both natural and man-made, such as within a body of a patient. The vessels, structures, lumens, and anatomy/tissue can include a blood vessel, as an artery or a vein of a patient's vascular system, including cardiac vasculature, peripheral vasculature, neural vasculature, renal vasculature, and/or any suitable lumen inside the body.

The system 100 can be referenced as an imaging system, ultrasound imaging system, intraluminal imaging system, and/or combinations thereof. Although the present disclosure refers to ICE catheters, any suitable intraluminal imaging or intraluminal sensing device is contemplated, such as an intravascular ultrasound (IVUS) device, an optical coherence tomography (OCT) device, an intracardiac echocardiography (ICE) device, a transesophageal echocardiography (TEE) device, an intravascular photoacoustic (IVPA) imaging device, pressure-sensing guidewire or catheter, flow-sensing guidewire or catheter, and/or any suitable internal imaging or sensing device. Intraluminal devices with flexible elongate members such as catheters, guide wires, and/or guide catheter are contemplated.

The system 100 may be utilized in a variety of applications such as transseptal punctures, left atrial appendage closures, atrial fibrillation ablation, and valve repairs, and can be used to image vessels and structures within a living body. Although the system 100 is described in the context of intraluminal imaging procedures, the system 100 is suitable for use with any catheterization procedure. In addition, the imaging assembly 102 may include any suitable physiological sensor or component for diagnostic, treatment, and/or therapy. For example, the imaging assembly can include an imaging component, an ablation component, a cutting component, a morcellation component, a pressure-sensing component, a flow-sensing component, a temperature-sensing component, and/or combinations thereof. In some embodiments, the intraluminal imaging system 100 is used for generating two-dimensional and three-dimensional images.

Referring back to Fig. 1, the PIM 131 may provide a physical and electrical connection between the ultrasound imaging device 110 and the clinical control and processing system 130. Some embodiments of the present disclosure omit the PIM 131. In other embodiments, the PIM 131 is communicatively interposed between the ultrasound imaging device 110 and the processing system or clinical workstation 130. In some instances, the PIM 131 can be referenced as a patient interface cable. For example, a proximal connector 209 of the ultrasound imaging device 110, a distal connector of the PIM, and/or a proximal connector of the PIM may be configured to couple the ultrasound imaging device 110, the PIM 131, and the control and processing system together mechanically and electrically. The system 100 may include may include a connector junction 111 comprising a proximal connector 125 of the ultrasound imaging device 110 and the distal connector of the PIM 131.

In some embodiments, the control and processing system 130 may include one or more computers, processors, and/or computer systems. The control and processing system 130 may also be referred to as a console. In some embodiments, the PIM 131 is in mechanical and electrical communication with the control and processing system 130, such that the electrical signals are transmitted the ultrasound imaging device 110 through the PIM 131 and to the control and processing system 130. The control and processing system 130 may include one or more processors and/or memory modules forming a processing circuit that may process the electrical signals and output a graphical representation of the imaging data on the monitor 132. One or more electrical conductors of the ultrasound imaging device 110 and PIM 131 may facilitate communication between the control and processing system 130 and the ultrasound imaging device 110. For example, a user of the control and processing system 130 may control imaging using the ultrasound imaging device 110 via a control interface 134 of the control and processing system 130. Electrical signals representative of commands from the control and processing system 130 may be transmitted to the ultrasound imaging device 110 via connectors and/or cables in the PIM 131 and the ultrasound imaging device 110. The control and processing system 130 may be transportable and may include wheels or other devices to facilitate easy transportation by a user.

In some embodiments, the one or more components of the ultrasound imaging device 110 may be disposable components. For example, a user, such as a physician, may obtain the catheter 101 and/or the ultrasound imaging device 110 in a sterilized packaging. In some embodiments, the ultrasound imaging device 110 may be discarded or disposed of after a single use. In other embodiments, the ultrasound imaging device 110 can be sterilized and/or reprocessed for more than one use. The PIM 131 may be a re-usable component that is used in multiple procedures. For example, the PIM 131 can be cleaned between individual procedures, such as being treated with disinfectants to kill bacteria. In some embodiments, the PIM 131 may not be required to be sterilized before a medical procedure. For example, the PIM 131 can be sufficiently spaced from the patient such that use of a non-sterile PIM 131 is safe for the patient. The sterile-nonsterile connection at the connector assembly 111 between the ultrasound imaging device 110 and the PIM 131 may allow for a safe operating environment while saving costs by allowing expensive equipment to be reused.

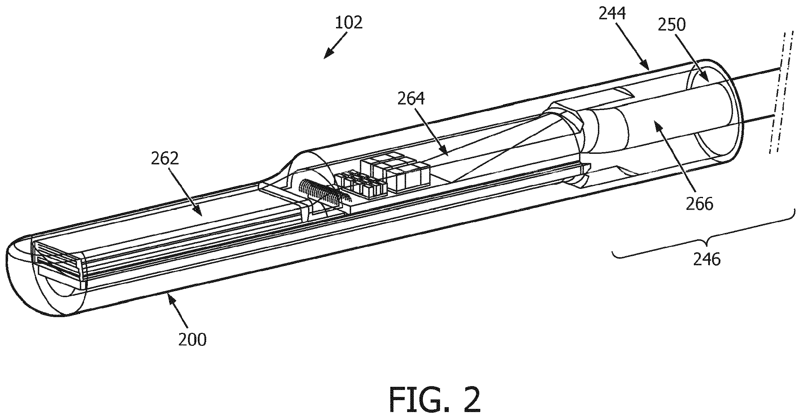

**Figure 2** is a perspective view of an example imaging assembly 102 positioned for coupling to a catheter 101 according to embodiments of the present disclosure. The imaging assembly 102 is illustrated with the imaging core 262 in position within the tip member 200. The imaging core 262 is coupled to an electrical cable 266 via an electrical interconnection 264. The electrical cable 266 extends through the alignment portion 244 and the interface portion 246 of the inner cavity 250. The electrical cable 266 can further extend through the flexible elongate member 101 as shown in Fig. 1.

The imaging core 262 can include an ultrasound transducer array. The imaging core 262 and/or the ultrasound transducer array are configured to obtain imaging data of the patient anatomy while positioned within a body lumen. The ultrasound transducer array can be an array of acoustic elements configured to emit ultrasound energy and receive echoes corresponding to the emitted ultrasound energy. In some instances, the array may include any number of ultrasound transducer elements. For example, the array can include between 2 acoustic elements and 10000 acoustic elements, including values such as 2 acoustic elements, 4 acoustic elements, acoustic elements, 64 acoustic elements, 128 acoustic elements, 500 acoustic elements, 812 acoustic elements, 3000 acoustic elements, 9000 acoustic elements, and/or other values both larger and smaller. In some instances, the transducer elements of the array may be arranged in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array can be configured to obtain one-dimensional, two-dimensional, and/or three-dimensional images of patient anatomy.

The ultrasound transducer elements may include piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array are in communication with (e.g., electrically coupled to) electronic circuitry. For example, the electronic circuitry can include one or more transducer control logic dies. The electronic circuitry can include one or more integrated circuits (IC), such as application specific integrated circuits (ASICs). In some embodiments, one or more of the ICs can include a microbeamformer (µBF). In other embodiments, one or more of the ICs includes a multiplexer circuit (MUX).

The configuration and structure of the tip member 200 described above provide several benefits such as safe and easy delivery for catheterization, improved tensile strength for steering or navigation, consistent or automatic alignment, and improved image quality. For example, the outer geometry of the tip member 200 is configured to provide smooth surfaces and smooth edges with small radii. The smooth edges reduce friction when the tip member 200 traverses a vessel during insertion. The smooth surfaces prevent tears and/or damage to tissue structures during the insertion. In addition, the smooth edges and smooth surfaces can facilitate crossing of a septum or other anatomical feature during a catheterization procedure. The material type and the wall thickness of the tip member 200 are selected to minimize acoustic distortion, attenuation, and/or reflection. The internal geometry of the tip member 200 is configured to facilitate alignment during manufacturing. The tip member 200 can also include other features, for example, a guidewire lumen, holes, or other geometry to accommodate additional devices or features such as pressure sensors, drug delivery mechanisms, and/or any suitable interventional features.

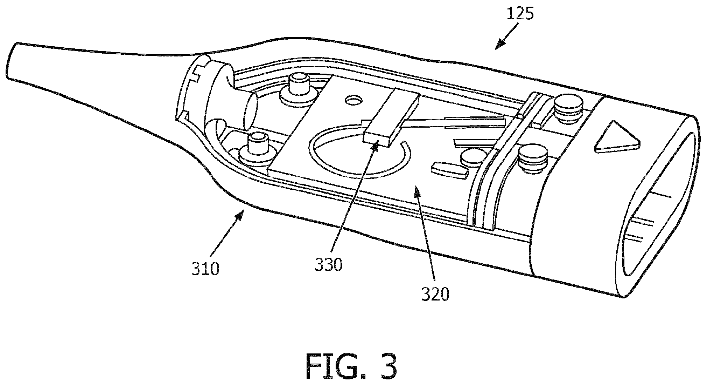

**Figure 3** shows the proximal portion of an example catheter cable attached to a connector 125 at the proximal portion of the catheter 101. The connector 125 includes a housing 310, a printed circuit board assembly (PCBA) 320 including one or more electronic components, and also including a non-volatile memory 330 such as a read-only memory (ROM), programmable read-only memory (PROM), electrically erasable read-only memory (EEPROM), magnetic or electronic random access memory (RAM), flash memory, etc.. The non-volatile memory 330 holds status variables that may be written by one or more of the manufacturer, the clinical workstation 130, or a reprocessor's workstation. In other embodiments, the EEPROM 330 may be located in other parts of the catheter 101, such as the handle 120 and/or the tip member 200. In generally, the memory 330 can be coupled to the catheter 101, such as a flexible elongate member of the catheter 101 (e.g., the catheter shaft and/or the cable 103).

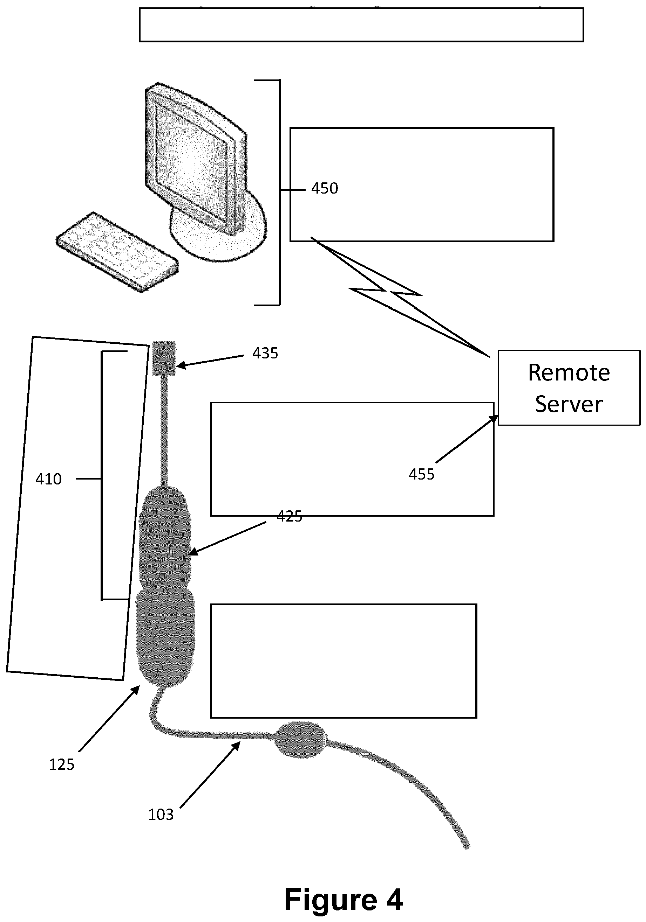

**Figure 4** is a schematic view of an example reprocessing workstation 450 according to at least one embodiment of the present disclosure. Visible are the cable 103 and connector 125 of the catheter 101, connected to the catheter connector 425 and workstation connector 435 of an adapter 410. In some embodiments, both the connector 125 and the connector 425 are proprietary interfaces designed to connect to one another, whereas workstation connector 435 is a standard connector such as a USB, micro-USB, Lightning, or FireWire connector that connects with the reprocessing workstation or reprocessor's workstation 450, thus enabling the reprocessing workstation 450 to read from and write to the non-volatile memory 330 within the connector 125 of the catheter 101. In other embodiments, the adaptor 410 is a wireless adaptor. In still other embodiments, the catheter connector 425 is built directly into the reprocessing workstation 450, such that the adaptor 410 is not required.

In some embodiments, the reprocessing station 450 runs a local application that interacts with the data on the non-volatile memory 330. In other embodiments, the reprocessing station runs a remote application (e.g., a web application) that enables a remote server 455 to interact with the data in the non-volatile memory 330. In still other applications, the reprocessing station 450 runs a local application but exchanges data with a remote server (e.g., a license confirmation status value of "Licensed" or "Not Licensed"). The reprocessing workstation 450 may communicate with the remote server through a wired or wireless connection. Wireless connection protocols may include Bluetooth, Wi-Fi, ZigBee, Li-Fi, or cellular data connections such as 2G/GSM, 3G/UMTS, 4G/LTE/WiMax, 5G, or other appropriate service or protocol. For example, a Bluetooth Low Energy (BLE) radio can be used to establish connectivity with a cloud service.

Depending on the implementation, the reprocessing workstation 450 may be a server, cloud computer, desktop computer, laptop computer, tablet computer, or handheld device, and may comprise any combination of general-purpose computing devices, reduced instruction set computing (RISC) devices, application-specific integrated circuits (ASICs), field programmable gate arrays (FPGAs), or other related logic devices, including mechanical and quantum computers. In some embodiments, the reprocessing workstation comprises a memory in which instructions or information are stored, and the processor operates based on the instructions or information. The memory may be co-located on the same board or chip with processing elements or else located external to a board or chip containing processing elements. The memory may comprise any combination of read-only memory (ROM), programmable read-only memory (PROM), electrically erasable read-only memory (EEPROM), magnetic or electronic random access memory (RAM), flash memory, disk or tape drive, or other related memory types.

In some embodiments, the reprocessing workstation is operated by a licensed or otherwise authorized reprocessing entity. Licenses may, for example, cover a specific number of devices (e.g., 100 devices), a specific number of reprocessings (e.g., 100 reprocessings), a specific window of time (e.g., the month of October), or may be ongoing or automatically renewing. In some embodiments, the reprocessing entity may also be an authorized distributor. In other embodiments, the reprocessing workstation is operated by the manufacturer, or by the clinical user of the catheter 101. In some embodiments, reprocessing may involve physical sterilization of the catheter (e.g., chemical, heat, or steam sterilization, or any combination thereof). In some embodiments, reprocessing may involve functional testing of the catheter 101 to ensure it is operating correctly.

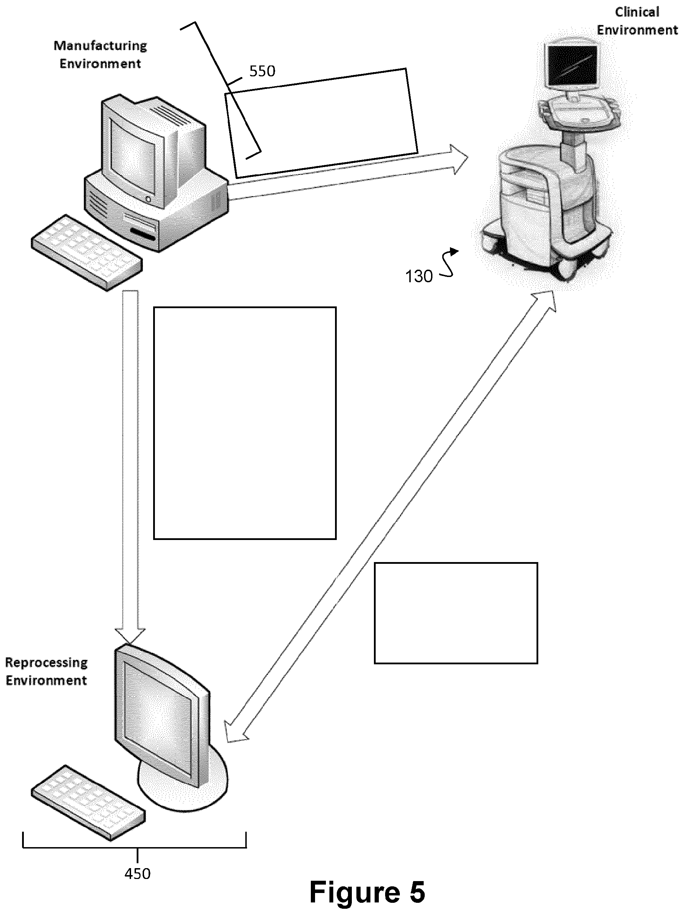

**Figure 5** is a schematic view of an example information flow between a manufacturing workstation 550, a clinical workstation 130, and a reprocessing workstation 450. Each of these workstations 550, 130, and 450 may comprise a processor circuit, a computer, a display, an input device (e.g., one or more of a keyboard, mouse, touchscreen, hard keys, soft keys, etc.), a console, or a mobile device. A usage tracking, disposable intraluminal imaging catheter 101 is manufactured, and its EEPROM 330 is written to by a manufacturing workstation 550. From there, the catheter 101 may be shipped directly to a clinical environment where it is plugged into a clinical workstation 130, which is also capable of reading and writing to the EEPROM 330, such that the use of the catheter 101 is recorded in the EEPROM 330 as described below. Alternatively, the catheter 101 may be shipped to a distributor/reprocessor, who later ships it to the clinical environment. Once the catheter 101 has been used, it is disconnected from the clinical workstation 130 and shipped to the distributor/reprocessor for reprocessing. The distributor/reprocessor connects the catheter 101 to the reprocessing workstation 450, which updates information in the EEPROM 330 such that the catheter 101 may be used again. The catheter 101 is then shipped back to the clinical environment (or a different clinical environment), where it may be used (e.g., plugged into the clinical workstation 130) again. The catheter 101 may be shipped back and forth between the clinical environment and the reprocessing environment as many times as the manufacturer has specified the catheter 101 may be used. For example, the manufacturer may designate a particular usage tracking, disposable intraluminal imaging catheter 101 to be a single-use device, or a multiple-use device (e.g., 5 uses), or a substantially reusable device (e.g., 100 or more uses).

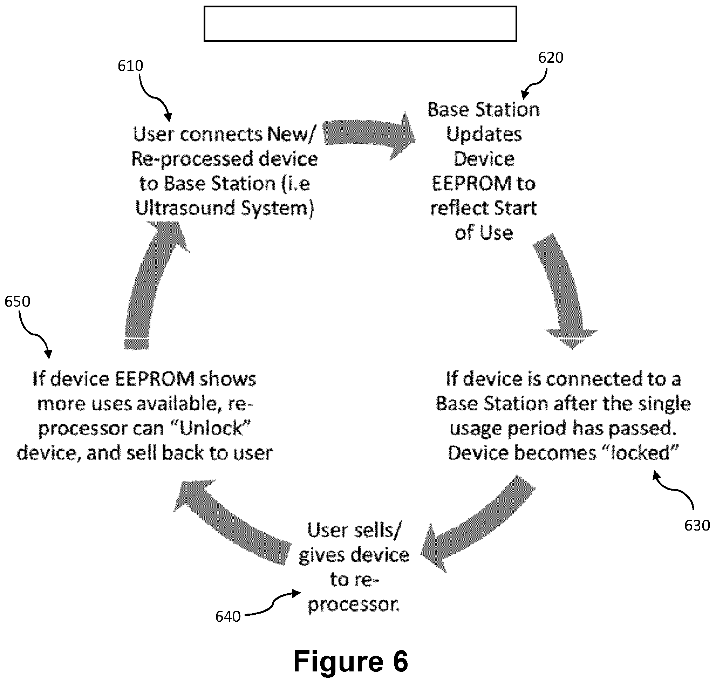

**Figure 6** is a flow diagram indicating exemplary movements of a usage tracking, disposable intraluminal imaging catheter 101 and changes to the device information stored thereon, according to at least one embodiment of the present disclosure.

In step 610 a user connects a catheter 101 to a clinical workstation or base station 130, such as an ultrasound system. The catheter 101 may be a new device shipped from the manufacturer or a distributor, or may be a reprocessed device shipped from the reprocessor.

In step 620, the clinical workstation or base station 130 updates information stored in the non-volatile memory 330 of the catheter 101, to reflect the start of a new use of the catheter 101. In an example, the use is limited to a certain duration or usage period (e.g., 8 hours), which is specified by a value stored in the non-volatile memory 330 of the catheter 101 as described below, and within this duration the catheter 101 may be connected to and disconnected from the clinical workstation or base station as many times as necessary or desired.

In step 630, the clinical workstation or base station 130 causes the catheter 101 to become "locked" if it becomes connected to, or remains connected to, the clinical workstation or base station 130 beyond the specified usage period. The locked state is specified by a value stored in the non-volatile memory 330 of the catheter 101 as described below. In an example, when the catheter 101 is in a locked state, the clinical workstation or base station 130 will not operate the catheter 101, and may display an error message or user instruction to have the catheter 101 reprocessed.

In step 640, the clinical user ships the catheter 101 to a reprocessor for reprocessing. Depending on the implementation, the device may be given, sold, loaned, rented, or otherwise transferred. In an example, the information stored in the non-volatile memory 330 of the catheter 101 includes a serial number, so the identity and ownership of the device may be readily tracked.

In step 650, the reprocessor connects the catheter 101 to a reprocessing workstation 450, which reads status variables stored within the non-volatile memory 330. If the status variables show that the catheter 101 is "locked" or "in use", but has more uses available (e.g., it has been used 3 times out of a maximum of 5 allowable uses), then the reprocessing workstation 450 writes new information into the status variables to unlock the device, as described below. The reprocessor may then give, sell, lend, rent, or lease the catheter 101 back to the clinical user, or a different clinical user. In some examples, the reprocessor cleans or sterilizes the catheter (e.g., using heat, chemicals, autoclave, pressure, steam, or boiling water) and/or tests the proper functioning of the catheter prior to returning it to clinical use. Operation of the catheter 101 then returns to step 610 until all available uses of the catheter 101 have been depleted.

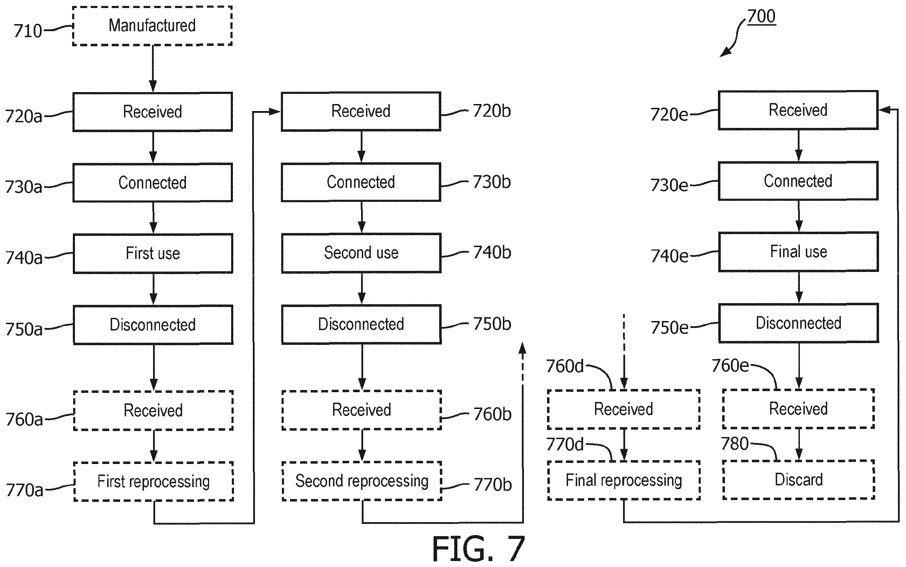

**Figure 7** is a more detailed flow diagram indicating exemplary steps in a method 700 for a usage tracking, disposable intraluminal imaging catheter 101 through multiple use and reprocessing cycles, according to at least one embodiment of the present disclosure.

In step 710, the catheter 101 is manufactured, at which time certain initial information is written into its non-volatile memory 330 as described below.

In step 720a, the catheter 101 is received by a clinical user. The catheter may have been shipped directly from the manufacturer, or may have passed through one or more dealers or distributors prior to reaching the clinical user.

In step 730a, the clinical user connects the catheter 101 to a clinical workstation 130. Beginning with this initial connection, the clinical workstation will then increment the usage counter within the non-volatile memory 330 of the catheter 101 (e.g., from 0 to 1). The catheter 101 then begins its first use, wherein the clinical workstation 130 marks it as "In Use" by writing status information to the non-volatile memory 330 of the catheter 101, and also writes a timestamp value into the non-volatile memory 330 indicating the date and time the use began, as described below.

In step 740a, the clinical user employs the device in a clinical procedure (e.g., an intraluminal ultrasound imaging procedure to evaluate anatomy). For example, use of the catheter 101 can include a processor circuit of the clinical workstation 130 controlling the catheter 101 to emit ultrasound energy and receive echoes reflected from the anatomy. The processor circuit of the clinical workstation 130 can process imaging data representative of the received echoes, and generate and display ultrasound images based on the imaging data. Throughout the period of use (including one or more disconnections and reconnections of the catheter 101 and clinical workstation 130), the clinical workstation 130 compares a current date and time against both the timestamp and a maximum duration of use. If the current date and time exceeds the sum of the timestamp and maximum duration of use, the clinical workstation 130 disables use of the catheter 101 and writes status information into the non-volatile memory 330 indicating the catheter 101 is now Locked, as described below. In some embodiments, in order to prevent interruption of ongoing medical procedures, this locking or disablement of the catheter is not enforced mid-use, but only after the catheter is disconnected from and reconnected to the system.

In step 750a, if the catheter 101 is Locked and will not be operated by the clinical workstation 130, the clinical workstation 130 displays an error message and instructs the clinical user to disconnect the catheter 101 from the clinical workstation 130 and send the catheter 101 to a reprocessor for reprocessing before it can be used again. Alternatively, if the clinical procedure is complete and the maximum duration of use has not yet expired, then the catheter 101 may be disconnected while it is still marked In Use, as described below. The catheter 101 may also be sent to a reprocessor in this state, and in some examples this may be the normal outcome. It should be noted that if a disconnected In Use catheter 101 is reconnected to a clinical workstation 130 after its maximum duration of use has expired, the clinical workstation 130 will mark the catheter 101 as Locked by writing the appropriate status information into the non-volatile memory 330. In this case, the catheter 101 is also sent to a reprocessor for reprocessing before the clinical workstation 130 will allow it to be used again.

In step 760a, the catheter 101 is received by the reprocessor. In this instance, the catheter 101 will most typically have been marked either In Use or Locked by the clinical workstation 130. In an example, upon receipt of the catheter 101 and prior to connecting the catheter 101 to a reprocessing workstation 450, the reprocessing entity (e.g., a corporation) will clean or sterilize the catheter 101 (e.g., with heat, chemicals, steam, or boiling water), and test its proper functioning.

In step 770a, the reprocessing entity connects the catheter 101 to a reprocessing workstation 450, which reads the status information stored in the non-volatile memory 330. If the catheter 101 is marked Unlocked (e.g., authorized for use), then the reprocessor has received it in error and may send it back to the clinical user, or a different clinical user, for normal clinical use. If the catheter 101 is marked either In Use or Locked, then the reprocessing workstation 450 will check to see whether the catheter 101 has any remaining uses. In the case of a single-use disposable catheter, no reprocessing is permitted (e.g., the number of allowable uses is one), and the reprocessing workstation will mark the catheter 101 as Locked, and may instruct the reprocessor to discard the catheter 101. If the catheter does have remaining uses, then the reprocessing workstation may mark the catheter as Unlocked so that it may be shipped back to the clinical user, or to a different clinical user. (The clinical station will then increment the usage counter within the non-volatile memory 330 of the catheter 101 the next time the catheter is reconnected to the clinical workstation).

The method 700 additional includes steps 720b - 770b, which are similar to steps 720a-770a and apply to subsequent connection, use, and reprocessing of the catheter 101. The clinical workstation 130 and reprocessor's workstation 450 will permit these steps to be repeated as many times as there are allowed uses of the catheter 101.

Step 770d represents the last permitted reprocessing of a catheter 101 (in this example, the fourth reprocessing, although it could be any number of reprocessings depending on the maximum number of uses specified in the status variables within the non-volatile memory 330). This is followed by step 770e, the final receipt of the catheter 101 by a clinical user, and 770e, the final connection of the catheter to a clinical workstation 130. In step 740e, the catheter 101 is used for the final time, and disconnected at step 750e with a status of either In Use or Locked.

In step 760e, the reprocessor receives the catheter 101 for the final time. When the device is connected to a reprocessing workstation 450, the reprocessing workstation detects that the catheter 101 has no remaining uses, and marks it Locked, and may set the number of uses equal to the maximum number of uses. In an example, the reprocessing workstation 450 may recommend discarding the catheter 101. In step 780, the usage tracking disposable intraluminal catheter is discarded (e.g., as trash or medical waste).

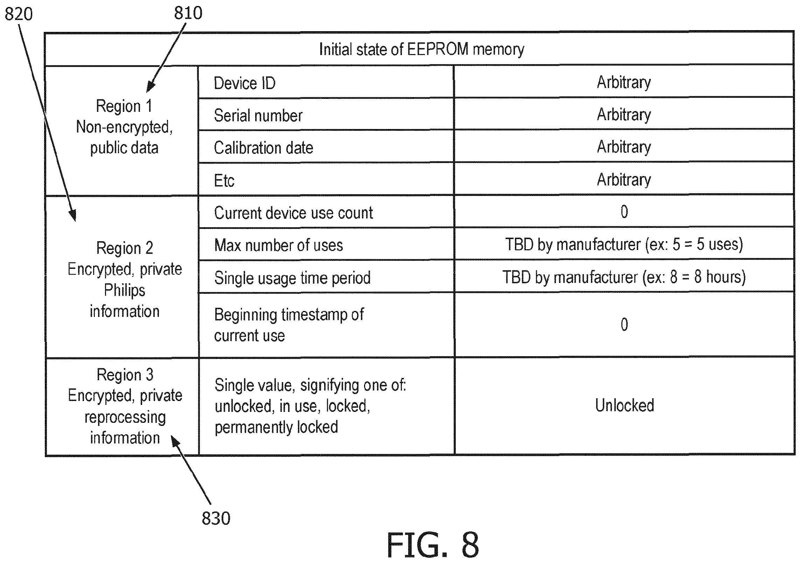

**Figure 8** shows an exemplary initial state of fields or status values within the non-volatile memory 330 of a usage tracking, disposable intraluminal catheter 101 when it leaves the manufacturer, according to at least one embodiment of the present disclosure. The non-volatile memory 330 is divided into three regions: a non-encrypted public data region 810 (also referred to as Region 1), an encrypted, private manufacturer information region 820 (also referred to as Region 2), and a private reprocessor information region 830 (also known as Region 3). It is understood that regions 810, 820, 830 refer to data that is accessible by one or more processors and stored according to the storage architecture (e.g., memory cells, bits, data blocks, etc.) within the memory 330. Regions 810, 820, 830 may but need not refer to a physical space within the memory 330. Accordingly, while data may belong to regions 810, 820, 830, they may be written in various physical spaces within the memory 330. In an example, the public data region 810 includes fields containing fixed, nonvariable information such as device ID or type, device serial number, device calibration date, manufacturing date, and other related information as necessary depending on the implementation. The information stored in the region 810 is public in that the data can be read for any workstation, including the clinical workstation 130 and the reprocessor workstation 450. In some embodiments, this information is unencrypted, although in other embodiments it may be encrypted by an appropriate method.

In some embodiments, the manufacturer manufactures all of the imaging device 101, the clinical workstation 130, and the reprocessor workstation 450. In other embodiments, the manufacturer manufactures the imaging device 101 such that it is configured for use with another manufacturer's clinical workstation 130.

The private manufacturer information region 820 (e.g., Region 2) includes fixed, non-variable information specified by the manufacturer, and not alterable through either the clinical workstation 130 or the reprocessor workstation 450. This information includes a usage counter, a maximum number of allowed uses (for example 5 uses, 100 uses, or 1000 uses), maximum elapsed time allowed per use, and other related information as necessary depending on the implementation. The private manufacturer information region 820 also includes variable information whose initial values are set by the manufacturer, but which can be updated dynamically by the clinical workstation 130. This information includes the current device use count (e.g., the number of times the catheter 101 has been used in a clinical procedure), and beginning timestamp of current use (e.g., the date and time when the catheter 101 is connected to a clinical workstation as described above in step 730a, 730b, etc. In an example, both of these variables are set to zero by default. The information may also include other variables as necessary, depending on the implementation. In some embodiments, the private manufacturer information region 820 is encrypted by an appropriate method, to discourage unauthorized parties from attempting to tamper with the information (e.g., to falsify information or to allow additional uses beyond the manufacturer specified maximum).

The private reprocessing information region 830 (also known as Region 3) is the only section of the non-volatile memory 330 that can be written to by the reprocessor workstation 450. This provides the security required such that the manufacturer can control reuse. The clinical workstation 130 can write to both the private manufacturing information region 820 (Region 2) and the private reprocessing information region 830 (Region 3). This provides access to variables in the non-volatile memory 330 through which the manufacturer can control reuse.

The private reprocessing information region 830 contains a status variable indicating whether the catheter 101 is Unlocked, In Use, or Locked. In an example, at the time of manufacture, this status variable is set to Unlocked by default. In some embodiments, the status variable may hold additional values including but not limited to Permanently Locked, Recalled, Damaged, Experimental, Demo, Permanently Unlocked, Error, or other values as necessary depending on the implementation. In some embodiments, the private reprocessing information region 830 is encrypted by an appropriate method, to discourage unauthorized parties from attempting to tamper with the information (e.g., to falsify information or to allow additional uses beyond the manufacturer specified maximum).

In general, the memory 330 can include two or more regions. In some embodiments, data is stored in the memory 330 in two regions. For example, the first region stores public data, and the second region store private, secured, and/or encrypted data. In some embodiments, the first region can be similar to the region 810 and store information such as device ID or type, device serial number, device calibration date, manufacturing date, and/or other related information as necessary depending on the implementation. In some embodiments, the second region can be similar to the regions 820 and 830, and store a usage counter, a maximum number of allowed uses (for example 5 uses, 100 uses, or 1000 uses), maximum elapsed time allowed per use, a status value representative of a status of the device, and/or indicating other related information as necessary depending on the implementation.

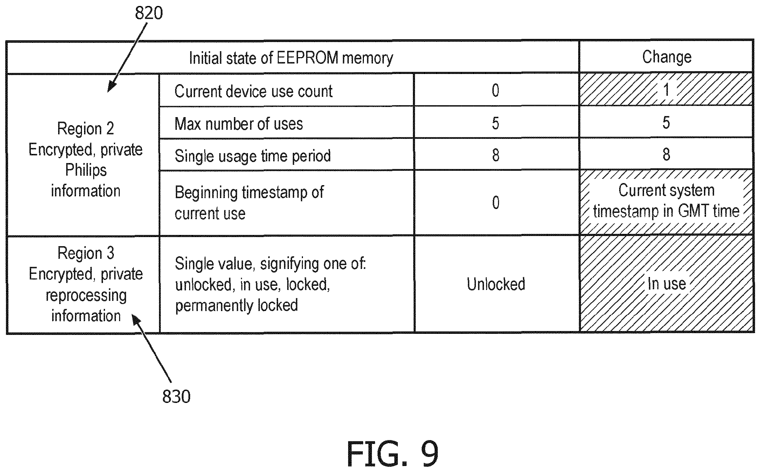

**Figure 9** shows an exemplary state of the non-volatile memory 330 during step 740a (e.g., when the catheter 101 is in use by the clinical workstation 130), according to at least one embodiment of the present disclosure.

The clinical workstation 130 reads the non-volatile memory 330 to verify whether the catheter 101 is permitted for use. For example, the clinical workstation 130 may compare current use count to the max number of uses to make sure that the current use value is less than the max value (e.g., that the catheter has at least one allowed use remaining). The clinical workstation is also seeing that the Region 3 value 830 is set to "unlocked" (e.g., that the catheter 101 is either new or has been correctly reprocessed by an authorized reprocessor). For example, if the number of uses of a catheter 101 exceeds the maximum number of allowed uses, then the clinical workstation 130 will refuse to operate the catheter 101, and the reprocessor workstation 450 will refuse to reprocess the catheter 101. If the time elapsed during the current use exceeds a maximum allowable duration, the clinical workstation will cease to operate the catheter 101. If Region 3 value 830 of the catheter is set to "locked", then the clinical workstation 130 will refuse to operate it, but the reprocessing station 450 will allow it to be reprocessed. Thus, after a permitted use, if a customer attempts to reuse the catheter 101 when it has not been reprocessed by an authorized reprocessor, one or more of the following will inform the clinical workstation 130 not to operate the device: (a) the use count being greater than the maximum allowed uses, (b) the elapsed time since activation being greater than the allowed single usage time period, or (c) the locked/unlocked status being set to "locked." In each of these cases, the system will issue an error message to the user, instructing the user to disconnect the catheter 101 and either dispose of it or return it to an authorized reprocessor, as appropriate.

The third column of Figure 9 shows the previous values from Figure 8 (e.g., the initial values set by the manufacturer), while the fourth column shows the values set during step 740a, with the changed values highlighted and the unchanged values not highlighted. In this example, the current device use count has been incremented from 0 to 1, the timestamp has been updated to the date and time (e.g., GMT time) that the current use began, and the reprocessing information status variable has been updated from Unlocked to In Use. Only memory regions 820 and 830 (e.g., Region 2 and Region 3) are shown in the figure, as in most implementations, the information in the public data memory region 810 (e.g., Region 1) cannot be changed by the clinical workstation 130. If the catheter 101 is disconnected from the clinical workstation 130 prior to the end of the single usage time period (e.g., the maximum allowable usage duration), the non-volatile memory 330 may be in the state shown in this figure when the device is sent to the reprocessor.

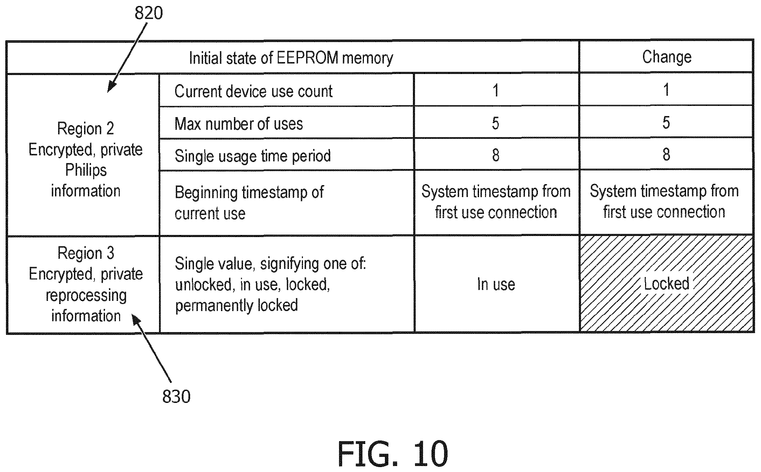

**Figure 10** shows the state of the non-volatile memory 330 at step 750a, according to at least one embodiment of the present disclosure. The third column shows the previous values from Figure 9 (e.g., the values during the first clinical use of the catheter 101), whereas the fourth column shows the values that occur if the catheter 101 remains connected to the clinical workstation 130 until the single usage time period has elapsed. In this case, the clinical workstation 130 updates the reprocessing information status variable to Locked. This is then the configuration that the non-volatile memory 330 will have when the catheter 101 is sent to the reprocessor.

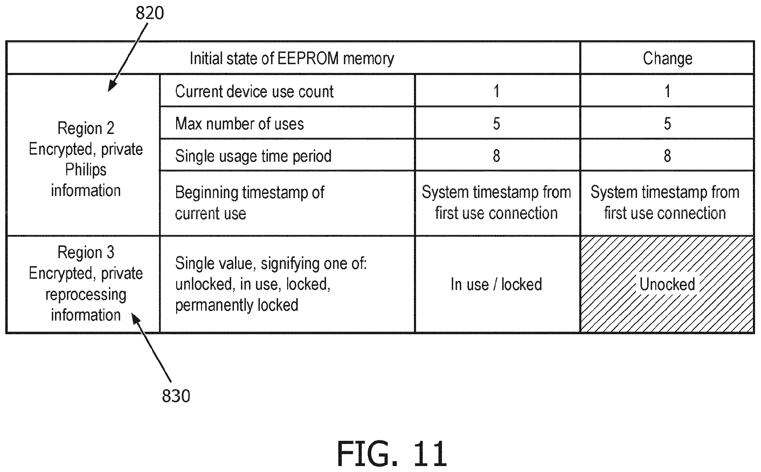

**Figure 11** shows the state of the non-volatile memory 330 after the processing in step 770a, according to at least one embodiment of the present disclosure. The third column shows the previous values from Figure 10 (e.g., the values following the first clinical use of the catheter 101), while the fourth column shows the values after reprocessing. As shown by the highlighting, the only change is that the reprocessing information status variable 830 is updated from a value of either In Use or Locked to a value of Unlocked.

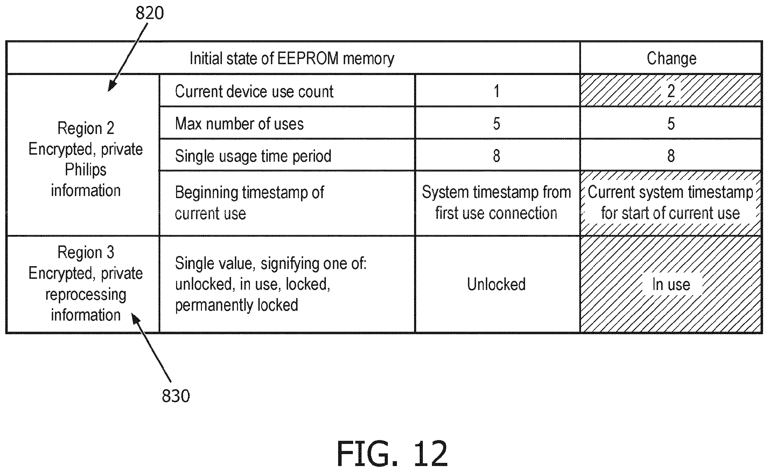

**Figure 12** shows the state of the non-volatile memory 330 during step 740b (e.g., during the second clinical use of the catheter 101) according to at least one embodiment of the present disclosure. As can be seen in the figure, the clinical workstation 130 updates the current device use count from 1 to 2, the timestamp to the time when the catheter 101 was connected to the clinical workstation 130, and the reprocessing information status variable 830 from Unlocked to In Use.

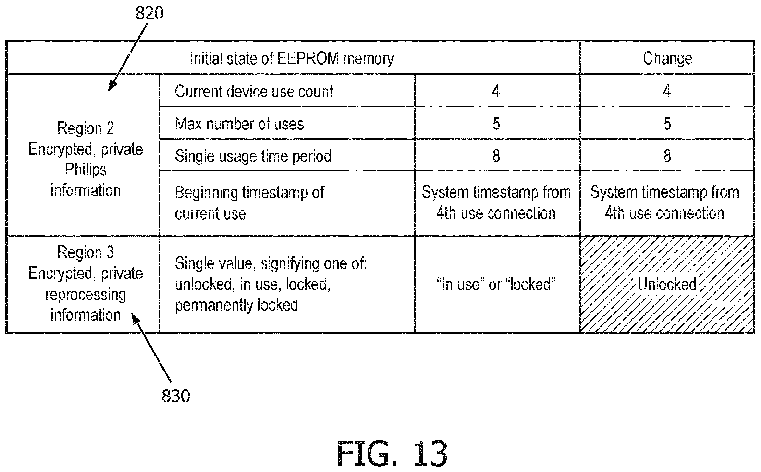

**Figure 13** shows the state of the non-volatile memory 330 after step 770d (e.g., following the final reprocessing of a catheter 101 with a maximum of 5 allowed uses), according to at least one embodiment of the present disclosure. In the example shown in the figure, the current device use count is 5, the max number of uses is 5, the single usage time period is 8 hours, the timestamp holds the time and date of the beginning of the catheter's 4^{th} clinical use, and the reprocessing information status variable 830 has been changed by the reprocessor workstation 450 from a value of either In Use or Locked to a value of Unlocked.

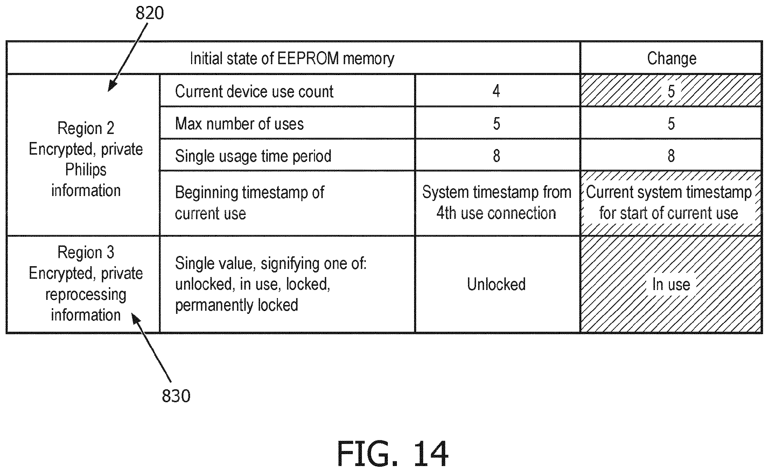

**Figure 14** shows the state of the non-volatile memory 330 during step 740e (e.g., during the final use of a catheter 101 with, in this example, 5 allowable uses), according to at least one embodiment of the present disclosure. In the example shown in the figure, the clinical workstation 130 has updated the current device use count from 4 to 5, and the timestamp to the date and time of the beginning of the current clinical use (e.g., when the catheter 101 was first connected to the clinical workstation 130 for this use). The clinical workstation 130 has also updated the reprocessing information status variable 830 from Unlocked to In Use.

As explained above, once the current system time shows that the single usage time period of 8 hours from the start of use, the clinical workstation 130 will cease operation of the catheter 101, and may recommend either disposal of the catheter 101 or else shipment of the catheter 101 to a reprocessing center for disposal. In some embodiments, in order to prevent interruption of ongoing medical procedures, the locking or other disablement of the catheter is not enforced mid-use, but only after the catheter is disconnected from and reconnected to the system.

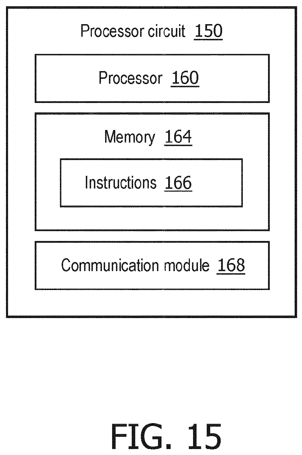

**Figure 15** is a schematic diagram of a processor circuit 150, according to embodiments of the present disclosure. The processor circuit 150 may be implemented in any of the clinical workstation 130, catheter 101, PIM 131 of Figure 1, the connector 125 of Figure 3, or the adapter 410 of Figure 4, or the manufacturer's workstation 550 or reprocessor workstation 450 of Figure 5, or other devices or workstations (e.g., third-party workstations) as necessary to implement the method. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The memory 164 may store instructions 166. The instructions 166 may include instructions that, when executed by the processor 160, cause the processor 160 to perform the operations described herein with reference to the workstations 130, 450, and 550 and/or the imaging device 101. Instructions 166 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 168 can include any electronic circuitry and/or logic circuitry to facilitate direct or indirect communication of data between the processor circuit 150, the imaging device 102, and/or the display 108. In that regard, the communication module 168 can be an input/output (I/O) device. In some instances, the communication module 168 facilitates direct or indirect communication between various elements of the processor circuit 150 and/or the workstations 130, 450 or 550. The communication module 168 may communicate through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols including but not limited to ISA, ATA, SCSI, PCI, IEEE-488, IEEE-1284, and other suitable protocols. Where appropriate, serial and parallel communications may be bridged by a UART, USART, or other appropriate subsystem.

A number of variations are possible on the examples and embodiments described above. For example, depending on the implementation, the incrementing of the current device use count within the private manufacturer region 820 of the non-volatile memory 330 may be performed by the clinical workstation 130 during a use, by the reprocessor's workstation 450 following a use, within the adapter 410 following a use, within the remote server 455 either during or following a use, within the catheter 101 itself either before, during, or following a use, or in any other location or step that achieves the disclosed result.

As another example, encryption of the non-volatile memory 330 may be a hash function (e.g., a hash of a device serial number), a symmetric key or public key encryption function, one-time pad, or any other appropriate encryption method to achieve the disclosed effects. Further, the non-volatile memory 330 may include a checksum value such that if any value in the non-volatile memory 330 is corrupted, hacked, erroneously written or altered, or otherwise changed except by the procedures, steps, methods, and devices described hereinabove, such change will be evident by a mismatch of the checksum. In some embodiments, to discourage unauthorized users from reverse-engineering the checksum algorithm, it may be advantageous to perform the checksum on the unencrypted version of the data in the non-volatile memory 330 and/or to encrypt the checksum itself. While encryption is mentioned as one way of securing the data on the memory 330, it is understood that any suitable security technique that prevents unauthorized access to particular data is contemplated. For example, password protection and/or software key-based security can be implemented.

The logical operations making up the embodiments of the technology described herein are referred to variously as operations, steps, objects, elements, components, or modules. Furthermore, it should be understood that these may be performed, assembled, or arranged in any order, unless explicitly claimed otherwise or a specific order is inherently necessitated by the claim language.

It should further be understood that the described technology may be employed in other types of intraluminal instruments, including both imaging and non-imaging sensors intended to study the vessels, lumens, or organs of a body. Further, the technology may be applied to fields completely separate from imaging/medicine. For example, it could apply to household items, construction tools, or any other reusable device that gets connected to a base system for use, and has a limited expected lifespan. Examples may include, but are not limited to light bulbs, electric toothbrush heads, electric buffer heads, and electric saw blades.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the usage tracking disposable intraluminal catheter, clinical workstation, manufacturer's workstation, or reprocessor's workstation.. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." Unless otherwise noted in the claims, stated values shall be interpreted as illustrative only and shall not be taken to be limiting.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure.

## Claims

1. An intraluminal imaging catheter (101), comprising:
a flexible elongate member configured to be positioned within a body lumen of a patient;
an ultrasound transducer array coupled to the flexible elongate member and configured to obtain imaging data while positioned within the body lumen; and
a memory (330) comprising a plurality of regions, wherein each of the plurality of regions is configured to store one or more values for one or more fields;
wherein the plurality of regions comprises a first region (810), wherein the one or more fields in the first region is configured to be accessed in read-only by any workstation adapted to connect to the intraluminal imaging catheter and comprises non-variable data information including at least one of a device identification, a serial number, or a manufacturing date;
wherein the plurality of regions comprises at least a second region (820), wherein one or more fields in the at least the second region is configured to be specified by a manufacturing workstation (550) only, and comprises at least one of a maximum number of allowed uses and maximum duration of allowed usage, wherein one or more other fields in the at least second region includes variable information, whose initial values are set by the manufacturing workstation (550) and which can be updated by at least one selected from the group of the catheter, a clinical workstation and a reprocessor workstation adapted to connect to the intraluminal imaging catheter, and wherein the one or more other fields including the variable information comprises at least one of a current use count and a beginning time stamp of current use;
wherein the plurality of regions further comprises a third region, wherein the one or more fields in the third region are configured to be written by at least one selected from the group of the the manufacturing workstation, the reprocessor workstation and the clinical workstation, and comprises a usage tracking status of the intraluminal imaging catheter.

2. The intraluminal imaging catheter of claim 1, wherein the one or more values in the second region and the one or more values in the third region are encrypted differently.

3. The intraluminal imaging catheter of claim 1, wherein the one or more values in the second region and the one or more values in the third region are encrypted based on the one or more values in the first region.

4. The intraluminal imaging catheter of claim 1, wherein the one or more values for the status of the intraluminal imaging catheter in the third region comprises:
the Unlocked value representative of authorization for the intraluminal imaging catheter to be used while connected to a clinical console;
the In-Use value representative of an initial connection of the intraluminal imaging catheter to the clinical console; or
the Locked value representative of a subsequent connection of the intraluminal imaging catheter to the clinical console.

5. The intraluminal imaging catheter of claim 4, wherein the one or more values for the status of the intraluminal imaging catheter in the third region comprises:
a permanently locked value representative of a connection of the intraluminal imaging catheter to the clinical console when use of the intraluminal imaging catheter exceeds the maximum number of uses.

6. The intraluminal imaging catheter of claim 1, further comprising:
a communication cable terminating at a connector, wherein the memory is disposed within the connector; and
a handle configured to control at least one of a position or an orientation of the ultrasound transducer array within the body lumen, wherein the handle is coupled to the communication cable and the flexible elongate member, and
wherein the ultrasound transducer array is configured for intracardiac echocardiography (ICE).

7. An intraluminal imaging system, comprising:
the intraluminal imaging catheter of any one of claims 1 to 6; and
one or more processor circuits in communication with the intraluminal imaging catheter and configured to:
operate the ultrasound transducer array to obtain the imaging data; and
write information into the non-volatile memory comprising:
a number of times the intraluminal imaging catheter may be used,
a number of times the intraluminal imaging catheter has been used; and
the status of the intraluminal imaging catheter,
wherein the one or more processor circuits will not operate the ultrasound transducer array if the status of the intraluminal imaging catheter is locked, or the number of times the intraluminal imaging catheter has been used is equal to or greater than the maximum number of uses.

8. The intraluminal imaging system of claim 7, wherein the one or more processor circuits is configured to set the status of the intraluminal imaging catheter to:
In-Use while the intraluminal imaging catheter is being used,
Locked after use of the intraluminal imaging catheter,
Unlocked during reprocessing of the intraluminal imaging catheter.

9. The intraluminal imaging system of claim 7, wherein at least a portion of the information written by the one or more processor circuits into the non-volatile memory is encrypted.

10. The intraluminal imaging system of claim 7, wherein the information written by the one or more processor circuits into the non-volatile memory further comprises:
a maximum duration for each use of the intraluminal imaging catheter; and
a time stamp indicating a start time of a use of the intraluminal imaging catheter,
wherein the one or more processor circuits comprises a timer, and
wherein once a difference between the timer and the time stamp equals or exceeds the maximum duration, the one or more processor circuits is configured to change the status of the intraluminal imaging catheter to Locked.

11. The intraluminal imaging system of claim 10, wherein, when the intraluminal imaging catheter is in use and the difference between the timer and the time stamp is less than the maximum duration, the intraluminal imaging catheter is configured to be repeatedly connected to and disconnected from a clinical workstation without triggering the one or more processor circuits to change the status of the intraluminal imaging catheter from In-Use to Locked.

12. An intraluminal imaging system, comprising:
the intraluminal imaging catheter of any one of claims 1 to 6,
wherein the memory comprises a non-volatile memory configured for communication with the manufacturer's workstation comprising a first processor circuit, the clinical workstation comprising a second processor circuit, and the reprocessor's workstation comprising a third processor circuit such that:
the manufacturer's workstation writes into the non-volatile memory a status value of Unlocked and a number of times the intraluminal imaging catheter may be used,
the clinical workstation operates the intraluminal imaging catheter and writes into the non-volatile memory:
a usage counter that increments every time the intraluminal imaging catheter is operated;
a status value of In-Use that replaces the status value of Unlocked while the intraluminal imaging catheter is being operated; and
a status value of Locked that replaces the status value of In-Use when a given time has elapsed since a start of the intraluminal imaging catheter being operated,
the reprocessor's workstation writes into the intraluminal imaging catheter a status value of Unlocked that replaces either or both of the status value of In-Use or the status value of Locked, and
the clinical workstation will not operate the intraluminal imaging catheter if the status value of Locked is in the non-volatile memory, or the usage counter contains a value larger than the number of times the intraluminal imaging catheter may be used.

13. The intraluminal imaging system of claim 12, further comprising:
the manufacturer's workstation, the clinical workstation, the reprocessor's workstation, wherein the reprocessor's workstation further comprises a license status value selected from Licensed or Not-Licensed.

## Patentansprüche

1. Intraluminaler Bildgebungskatheter (101), umfassend:
ein flexibles, längliches Element, das so konfiguriert ist, dass es in einem Körperlumen eines Patienten positioniert werden kann;
ein Ultraschallwandlerarray, das mit dem flexiblen, länglichen Element gekoppelt und so konfiguriert ist, dass es Bilddaten erhält, während es im Körperlumen positioniert ist; und
einen Speicher (330), der eine Vielzahl von Bereichen umfasst, wobei jeder der Vielzahl von Bereichen so konfiguriert ist, dass er einen oder mehrere Werte für ein oder mehrere Felder speichert;
wobei die Vielzahl der Bereiche einen ersten Bereich (810) umfasst,
wobei das eine oder die mehreren Felder im ersten Bereich so konfiguriert sind, dass sie von jeder Arbeitsstation, die an die Verbindung mit dem intraluminalen Bildgebungskatheter angepasst ist, schreibgeschützt aufgerufen werden können und nicht-variable Dateninformationen umfassen, die mindestens eines von einer Vorrichtungsidentifikation, einer Seriennummer oder einem Herstellungsdatum einschließen;
wobei die Vielzahl der Bereiche mindestens einen zweiten Bereich (820) umfasst,
wobei ein oder mehrere Felder in dem mindestens zweiten Bereich so konfiguriert sind, dass es nur von einer Herstellungsarbeitsstation (550) spezifiziert werden können und mindestens eines von einer maximalen Anzahl zulässiger Verwendungen und einer maximalen Dauer der zulässigen Verwendung umfassen,
wobei ein oder mehrere andere Felder in dem mindestens zweiten Bereich variable Informationen einschließen, deren Anfangswerte von der Herstellungsarbeitsstation (550) festgelegt werden und die von mindestens einer, ausgewählt aus der Gruppe des Katheters, einer klinischen Arbeitsstation und einer Aufbereitungsarbeitsstation, die an die Verbindung mit dem intraluminalen Bildgebungskatheter angepasst ist, aktualisiert werden können, und wobei das eine oder die mehreren anderen Felder, die die variablen Informationen einschließen, mindestens eines von einem aktuellen Verwendungszähler und einen Startzeitstempel der aktuellen Verwendung umfassen;
wobei die Vielzahl von Bereichen weiter einen dritten Bereich umfasst, wobei das eine oder die mehreren Felder im dritten Bereich so konfiguriert sind, dass sie von mindestens einer, ausgewählt aus der Gruppe der Herstellungsarbeitsstation, der Aufbereitungsarbeitsstation und der klinischen Arbeitsstation, beschrieben werden können und einen Verwendungsstatus des intraluminalen Bildgebungskatheters umfassen.

2. Intraluminaler Bildgebungskatheter nach Anspruch 1, wobei der eine oder die mehreren Werte im zweiten Bereich und der eine oder die mehreren Werte im dritten Bereich unterschiedlich verschlüsselt werden.

3. Intraluminaler Bildgebungskatheter nach Anspruch 1, wobei der eine oder die mehreren Werte im zweiten Bereich und der eine oder die mehreren Werte im dritten Bereich auf der Grundlage des einen oder der mehreren Werte im ersten Bereich verschlüsselt werden.

4. Intraluminaler Bildgebungskatheter nach Anspruch 1, wobei der eine oder die mehreren Werte für den Status des intraluminalen Bildgebungskatheters im dritten Bereich Folgendes umfassen:
den Wert "Entsperrt", der die Autorisierung zur Verwendung des intraluminalen Bildgebungskatheters bei Verbindung mit einer klinischen Konsole darstellt;
den Wert "Im Gebrauch", der eine erstmalige Verbindung des intraluminalen Bildgebungskatheters mit der klinischen Konsole darstellt; oder
den Wert "Gesperrt", der eine nachfolgende Verbindung des intraluminalen Bildgebungskatheters mit der klinischen Konsole darstellt.

5. Intraluminaler Bildgebungskatheter nach Anspruch 4, wobei der eine oder die mehreren Werte für den Status des intraluminalen Bildgebungskatheters im dritten Bereich Folgendes umfassen:
einen dauerhaft gesperrten Wert, der eine Verbindung des intraluminalen Bildgebungskatheters mit der klinischen Konsole darstellt, wenn die Verwendung des intraluminalen Bildgebungskatheters die maximale Anzahl an Verwendungen überschreitet.

6. Intraluminaler Ultraschallbildgebungskatheter nach Anspruch 1, weiter umfassend:
ein Kommunikationskabel, das an einem Stecker endet, wobei der Speicher innerhalb des Steckers angeordnet ist; und
einen Griff, der so konfiguriert ist, dass er mindestens eine von einer Position oder einer Ausrichtung des Ultraschallwandlerarrays im Körperlumen steuert, wobei der Griff mit dem Kommunikationskabel und dem flexiblen, länglichen Element gekoppelt ist, und
wobei das Ultraschallwandlerarray für die intrakardiale Echokardiographie (ICE) konfiguriert ist.

7. Intraluminales Bildgebungssystem, das Folgendes umfasst:
den intraluminalen Bildgebungskatheter nach einem der Ansprüche 1 bis 6; und
eine oder mehrere Prozessorschaltungen, die mit dem intraluminalen Bildgebungskatheter kommunizieren und konfiguriert sind zum:
Betreiben des Ultraschallwandlerarrays, um die Bilddaten zu erhalten; und
Schreiben von Informationen in den nichtflüchtigen Speicher, umfassend:
eine Anzahl von zulässigen Verwendungen des intraluminalen Bildgebungskatheters,
eine Anzahl von Malen, in denen der intraluminale Bildgebungskatheter verwendet wurde; und
den Status des intraluminalen Bildgebungskatheters,
wobei die eine oder die mehreren Prozessorschaltungen das Ultraschallwandlerarray nicht betreiben, wenn der Status des intraluminalen Bildgebungskatheters gesperrt ist oder die Anzahl der Verwendungen des intraluminalen Bildgebungskatheters gleich oder größer als die maximale Anzahl der Verwendungen ist.

8. Intraluminaler Bildgebungssystem nach Anspruch 7, wobei die eine oder mehreren Prozessorschaltungen so konfiguriert sind, dass sie den Status des intraluminalen Bildgebungskatheters wie folgt einstellen:
"Im Gebrauch", während der intraluminale Bildgebungskatheter verwendet wird.
"Gesperrt" nach Verwendung des intraluminalen Bildgebungskatheters.
"Entsperrt" während der Aufbereitung des intraluminalen Bildgebungskatheters.

9. Intraluminales Bildgebungssystem nach Anspruch 7, wobei mindestens ein Abschnitt der von der einen oder den mehreren Prozessorschaltungen in den nichtflüchtigen Speicher geschriebenen Informationen verschlüsselt ist.

10. Intraluminales Bildgebungssystem nach Anspruch 7, wobei die von der einen oder den mehreren Prozessorschaltungen in den nichtflüchtigen Speicher geschriebenen Informationen weiter Folgendes umfassen:
eine maximale Dauer für jede Verwendung des intraluminalen Bildgebungskatheters; und
einen Zeitstempel, der den Beginn der Verwendung des intraluminalen Bildgebungskatheters angibt,
wobei die eine oder die mehreren Prozessorschaltungen einen Timer umfassen, und
wobei, sobald die Differenz zwischen dem Timer und dem Zeitstempel die maximale Dauer erreicht oder überschreitet, die eine oder die mehreren Prozessorschaltungen so konfiguriert sind, dass sie den Status des intraluminalen Bildgebungskatheters auf "Gesperrt" ändern.

11. Intraluminales Bildgebungssystem nach Anspruch 10, wobei, wenn der intraluminale Bildgebungskatheter im Gebrauch ist und die Differenz zwischen dem Timer und dem Zeitstempel kleiner als die maximale Dauer ist, der intraluminale Bildgebungskatheter so konfiguriert ist, dass er wiederholt mit einer klinischen Arbeitsstation verbunden und von dieser getrennt werden kann, ohne dass die eine oder die mehreren Prozessorschaltungen den Status des intraluminalen Bildgebungskatheters von "Im Gebrauch" auf "Gesperrt" ändern.

12. Intraluminales Bildgebungssystem, das Folgendes umfasst:
den intraluminalen Bildgebungskatheter nach einem der Ansprüche 1 bis 6,
wobei der Speicher einen nichtflüchtigen Speicher umfasst, der für die Kommunikation mit der Herstellerarbeitsstation, die eine erste Prozessorschaltung umfasst, der klinischen Arbeitsstation, die eine zweite Prozessorschaltung umfasst, und der Aufbereitungsarbeitsstation, die eine dritte Prozessorschaltung umfasst, konfiguriert ist, sodass:
die Herstellerarbeitsstation in den nichtflüchtigen Speicher einen Statuswert "Entsperrt" und eine Anzahl von zulässigen Verwendungen des intraluminalen Bildgebungskatheters schreibt,
die klinische Arbeitsstation den intraluminalen Bildgebungskatheter betreibt und in den nichtflüchtigen Speicher Folgendes schreibt:
einen Verwendungszähler, der sich bei jedem Betrieb des intraluminalen Bildgebungskatheters erhöht;
einen Statuswert "Im Gebrauch", der den Statuswert "Entsperrt" ersetzt, während der intraluminale Bildgebungskatheter im Betrieb ist; und
einen Statuswert "Gesperrt", der den Statuswert "Im Gebrauch" ersetzt, wenn seit einem Betriebsbeginn des intraluminalen Bildgebungskatheters eine vorgegebene Zeit verstrichen ist;
die Aufbereitungsarbeitsstation in den intraluminalen Bildgebungskatheter einen Statuswert "Entsperrt" schreibt, der entweder den Statuswert "Im Gebrauch" oder den Statuswert "Gesperrt" oder beide ersetzt, und
die klinische Arbeitsstation den intraluminalen Bildgebungskatheter nicht betreibt, wenn im nichtflüchtigen Speicher der Statuswert "Gesperrt" gespeichert ist oder der Verwendungszähler einen Wert enthält, der größer ist als die Anzahl von zulässigen Verwendungen des intraluminalen Bildgebungskatheters.

13. Intraluminales Bildgebungssystem nach Anspruch 12, weiter umfassend:
die Herstellerarbeitsstation, die klinische Arbeitsstation, die Aufbereitungsarbeitsstation, wobei die Aufbereitungsarbeitsstation einen Lizenzstatuswert umfasst, der aus "Lizenziert" oder "Nicht lizenziert" ausgewählt ist.

## Revendications

1. Cathéter d'imagerie intraluminale (101), comprenant :
un élément allongé flexible configuré pour être positionné dans une lumière corporelle d'un patient ;
un réseau de transducteurs à ultrasons couplé à l'élément allongé flexible et configuré pour obtenir des données d'imagerie lorsqu'il est positionné à l'intérieur de la lumière corporelle ; et
une mémoire (330) comprenant une pluralité de régions, dans laquelle chacune des régions est configurée pour stocker une ou plusieurs valeurs pour un ou plusieurs champs ;
dans lequel la pluralité de régions comprend une première région (810),
dans lequel l'un ou les plusieurs champs de la première région sont configurés pour être accessibles en lecture seule par tout poste de travail adapté pour se connecter au cathéter d'imagerie intraluminale et comprennent des informations de données non variables, incluant au moins l'un d'un dispositif d'identification, d'un numéro de série ou d'une date de fabrication ;
dans lequel la pluralité de régions comprend au moins une deuxième région (820),
dans lequel un ou plusieurs champs de la au moins deuxième région sont configurés pour être spécifiés uniquement par un poste de travail du fabricant (550), et comprennent au moins l'un d'un nombre maximal d'utilisations autorisées et d'une durée maximale d'utilisation autorisée,
dans lequel un ou plusieurs autres champs dans au moins la deuxième région inclut des informations variables, dont les valeurs initiales sont définies par le poste de travail du fabricant (550) et qui peuvent être mises à jour par au moins l'un sélectionné dans le groupe constitué du cathéter, d'un poste de travail clinique et d'un poste de travail de retraitement adapté pour se connecter au cathéter d'imagerie intraluminale, et dans lequel l'un ou les plusieurs autres champs incluant les informations variables comprennent au moins l'un d'un nombre d'utilisations actuelles et d'un horodatage de début d'utilisation actuelle ;
dans lequel la pluralité de régions comprend en outre une troisième région, dans lequel l'un ou les plusieurs champs de la troisième région sont configurés pour être écrits par au moins l'un sélectionné dans le groupe constitué du poste de travail du fabricant, du poste de travail de retraitement et du poste de travail clinique, et comprend un état de suivi d'utilisation du cathéter d'imagerie intraluminale.

2. Cathéter d'imagerie intraluminale selon la revendication 1, dans lequel l'une ou les plusieurs valeurs dans la deuxième région et la ou les plusieurs valeurs de la troisième région sont cryptées différemment.

3. Cathéter d'imagerie intraluminale selon la revendication 1, dans lequel l'une ou les plusieurs valeurs de la deuxième région et l'une ou les plusieurs valeurs de la troisième région sont cryptées en fonction de l'une ou des plusieurs valeurs de la première région.

4. Cathéter d'imagerie intraluminale selon la revendication 1, dans lequel l'une ou les plusieurs valeurs relatives à l'état du cathéter d'imagerie intraluminale dans la troisième région comprennent :
la valeur de l'état "Déverrouillé" représentative de l'autorisation d'utiliser le cathéter d'imagerie intraluminale lorsqu'il est connecté à une console clinique ;
la valeur de l'état "En cours d'utilisation" représentative d'une connexion initiale du cathéter d'imagerie intraluminale à la console clinique ; ou
la valeur de l'état "Verrouillé" représentative d'une connexion ultérieure du cathéter d'imagerie intraluminale à la console clinique.

5. Cathéter d'imagerie intraluminale selon la revendication 4, dans lequel l'une ou les plusieurs valeurs relatives à l'état du cathéter d'imagerie intraluminale dans la troisième région comprennent :
une valeur de l'état "Verrouillé" en permanence représentative d'une connexion du cathéter d'imagerie intraluminale à la console clinique lorsque l'utilisation du cathéter d'imagerie intraluminale dépasse le nombre maximal d'utilisations.

6. Cathéter d'imagerie intraluminale selon la revendication 1, comprenant en outre :
un câble de communication se terminant par un connecteur, dans lequel la mémoire est disposée à l'intérieur du connecteur ; et
une poignée configurée pour contrôler au moins la position ou l'orientation du réseau de transducteurs à ultrasons à l'intérieur de la lumière corporelle, dans lequel poignée est reliée au câble de communication et à l'élément flexible allongé, et
dans lequel le réseau de transducteurs à ultrasons est configuré pour l'échocardiographie intracardiaque (EIC).

7. Système d'imagerie intraluminale, comprenant :
le cathéter d'imagerie intraluminale selon l'une quelconque des revendications 1 à 6 ; et
un ou plusieurs circuits de processeur en communication avec le cathéter d'imagerie intraluminale et configurés pour :
actionner le réseau de transducteurs à ultrasons pour obtenir les données d'imagerie ; et
écrire des informations dans la mémoire non volatile comprenant :
un nombre de fois le cathéter d'imagerie intraluminale pouvant être utilisé.
un nombre de fois le cathéter d'imagerie intraluminale ayant été utilsé ; et
l'état du cathéter d'imagerie intraluminale,
dans lequel l'un ou les plusieurs circuits de processeur ne feront pas fonctionner le réseau de transducteurs à ultrasons si l'état du cathéter d'imagerie intraluminale est verrouillé ou si le nombre de fois le cathéter d'imagerie intraluminale ayant été utilisé est égal ou supérieur au nombre maximal d'utilisations.

8. Système d'imagerie intraluminale selon la revendication 7, dans lequel l'un ou les plusieurs circuits de processeur sont configurés pour définir l'état du cathéter d'imagerie intraluminale pour :
passer à l'état "En cours d'utilisation" pendant l'utilisation du cathéter d'imagerie intraluminale.
passer à l'état "Verrouillé" après utilisation du cathéter d'imagerie intraluminale.
passer à l'état "Déverrouillé " lors du retraitement du cathéter d'imagerie intraluminale.

9. Système d'imagerie intraluminale selon la revendication 7, dans lequel au moins une partie des informations écrites par l'un ou les plusieurs circuits de processeur dans la mémoire non volatile est cryptée.

10. Système d'imagerie intraluminale selon la revendication 7, dans lequel les informations écrites par l'un ou les plusieurs circuits de processeur dans la mémoire non volatile comprennent en outre :
une durée maximale pour chaque utilisation du cathéter d'imagerie intraluminale ; et
un horodatage indiquant l'heure de début d'utilisation du cathéter d'imagerie intraluminale,
dans lequel l'un ou les plusieurs circuits de processeur comprennent une minuterie, et
dans lequel, dès lors qu'une différence entre la minuterie et l'horodatage atteint ou dépasse la durée maximale, l'un ou les plusieurs circuits de processeur sont configurés pour changer l'état du cathéter d'imagerie intraluminale à l'état 'Verrouillé".

11. Système d'imagerie intraluminale selon la revendication 10, dans lequel, lorsque le cathéter d'imagerie intraluminale est en cours d'utilisation et que la différence entre la minuterie et l'horodatage est inférieure à la durée maximale, le cathéter d'imagerie intraluminale est configuré pour être connecté et déconnecté de manière répétée d'un poste de travail clinique sans déclencher l'un ou les plusieurs circuits de processeur pour changer l'état du cathéter d'imagerie intraluminale de l'état 'En cours d'utilisation" à l'état "Verrouillé".

12. Système d'imagerie intraluminale, comprenant :
le cathéter d'imagerie intraluminale selon l'une quelconque des revendications 1 à 6,
dans lequel la mémoire comprend une mémoire non volatile configurée pour communiquer avec le poste de travail du fabricant comprenant un premier circuit de processeur, le poste de travail clinique comprenant un deuxième circuit de processeur et le poste de travail du retraitement comprenant un troisième circuit de processeur, de sorte que :
le poste de travail du fabricant écrit dans la mémoire non volatile une valeur d'état « Déverrouillé » et le nombre de fois le cathéter d'imagerie intraluminale pouvant être utilisé.
le poste de travail clinique actionne le cathéter d'imagerie intraluminale et écrit dans la mémoire non volatile :
un compteur d'utilisations qui s'incrémente à chaque utilisation du cathéter d'imagerie intraluminale ;
une valeur d'état "En cours d'utilisation " qui remplace la valeur d'état "Déverrouillé " pendant le fonctionnement du cathéter d'imagerie intraluminale ; et
une valeur d'état "Verrouillé " qui remplace la valeur d'état " En cours d'utilisation " lorsqu'un délai donné s'est écoulé depuis le début du fonctionnement du cathéter d'imagerie intraluminale,
le poste de travail du retraitement inscrit dans le cathéter d'imagerie intraluminale une valeur d'état "Déverrouillé" qui remplace soit la valeur d'état "En cours d'utilisation" ou la valeur d'état "Verrouillé" soit les deux.
le poste de travail clinique ne fera pas fonctionner le cathéter d'imagerie intraluminale si la valeur d'état "Verrouillé" se trouve dans la mémoire non volatile, ou si le compteur d'utilisations contient une valeur supérieure au nombre de fois le cathéter d'imagerie intraluminale pouvant être utilisé.

13. Système d'imagerie intraluminale selon la revendication 12, comprenant en outre :
le poste de travail du fabricant, le poste de travail clinique, le poste de travail du retraitement, dans lequel le poste de travail de retraitement comprend en outre une valeur de statut de licence sélectionnée parmi "Autorisé " ou " Non autorisé ".
